# EUROPEAN PATENT SPECIFICATION

(11) **EP 3 706 680 B1**
(45) Date of publication and mention of the grant of the patent: **15.05.2024**
(21) Application number: 18836869.0
(22) Date of filing: 13.12.2018
(51) Int. Cl.: A61F 9/007

(54) **ULTRAVIOLET LASER VITRECTOMY PROBE**
VITREKTOMIESONDE MIT ULTRAVIOLETTEM LASER
SONDE DE VITRECTOMIE À LASER ULTRAVIOLET

(30) Priority: 04.01.2018 US 201862613715 P
(43) Date of publication of application: 16.09.2020
(73) Proprietor: Alcon Inc., 1701 Fribourg (CH)
(72) Inventor: BACHER, Gerald David, Lake Forest, California 92630 (US); OVCHINNIKOV, Mikhail, Lake Forest, California 92630 (US); BOR, Zsolt, Lake Forest, California 92630 (US)
(74) Representative: Murgitroyd & Company
(86) International application number: PCT/IB2018/060050
(87) International publication number: WO 2019/135127

(56) References cited:
- US-A- 5 478 338
- US-A1- 2002 123 744
- US-A1- 2009 171 326
- US-A1- 2014 364 870
- US-A1- 2016 120 699

## Description

### TECHNICAL FIELD

The present disclosure is directed to systems and instruments for use in medical procedures, and more particularly, to systems for vitrectomy procedures.

### BACKGROUND

Vitreo-retinal procedures are commonly performed within the posterior chamber of the human eye to treat various conditions of the posterior segment of the eye. For example, vitreo-retinal procedures may treat conditions such as age-related macular degeneration (AMD), diabetic retinopathy and diabetic vitreous hemorrhage, macular hole, retinal detachment, epiretinal membrane, cytomegalovirus (CMV) retinitis, and many other ophthalmic conditions.

Such procedures frequently require the cutting and removal of portions of the vitreous humor from the posterior segment of the eye. The vitreous humor is comprised of microscopic fibers or strands within the posterior chamber. A surgeon may perform vitreo-retinal procedures with a microscope and special lenses designed to provide a clear image of the posterior segment. Several tiny incisions are typically made on the sclera at the pars plana. The surgeon inserts microsurgical instruments through the incisions, including a vitrectomy probe to cut and remove the strands of the vitreous body.

Various vitrectomy probes are disclosed, for example, in U.S. Patent No. 9,615,969, U.S. Patent No. 9,381,114, and U.S. Patent No. 9,757,273.

In certain prior vitrectomy probes, the instrument may include an external tube with a port or hole in the tube, for example in the side of the tube. The instrument may further include an internal tube within the external tube, the internal tube having a cutting surface at its distal edge. Suction is applied to draw the vitreous fibers into the port of the external tube, while the internal tube reciprocates at high speed. As the internal tube approaches and passes by the port, the action of the cutting edge of the internal tube against the vitreous fibers cuts or breaks the vitreous fibers such that the vitreous material can be suctioned away and removed.

In ultrasonic vitrectomy probes, the instrument uses ultrasonic action to cut or break the vitreous fibers. More specifically, the high speed ultrasonic oscillation of the instrument causes the vitreous fibers to cut or break such that the vitreous material can be suctioned away and removed.

The removal of vitreous fibers is a sensitive procedure, which must be performed efficiently and without damage to the retina or other parts of the eye. The ends of the vitreous fibers are attached directly to the retina, and pulling on these fibers can potentially detach or tear the retina. Accordingly, it is desired to improve upon existing vitrectomy probes.
Reference is made to US 2014/0364870, US 2009/0171326, US5478338 and US2016/0120699 which have been cited as relating to the state of the art of various ophthalmic systems.

### SUMMARY

It will be appreciated that the scope is in accordance with the claims. Accordingly, there is provided a vitrectomy probe as defined in claim 1. Further features are provided in accordance with the dependent claims. The specification may include description of arrangements outside the scope of the claims provided as background and to assist in understanding the invention.

One or more arrangements of the present disclosure include a vitrectomy probe for treating an eye of a patient, the vitrectomy probe including a body, and a disruption element extending from the body. The disruption element including a needle comprising a main lumen and a side port at a distal end. The disruption element further includes an ultraviolet (UV) optical fiber projecting a UV laser beam for irradiating an area proximate the port. The disruption element further comprising an actuator for controlling movement of the UV optical fiber. The disruption element further including a UV light source for generating the UV laser beam in a spectral range of approximately 190 - 220 nanometers and the UV optical fiber is mechanically moved within the fiber lumen such that the UV laser beam being projected from the UV optical fiber scans across the side port to sever vitreous.

In an arrangement (not presently claimed), a method for operating a vitrectomy probe is described, the method including projecting a UV laser beam from an UV optical fiber, the UV laser beam directed across a port of a needle of the vitrectomy probe. The method may further include receiving a vitreous material through the port, wherein the UV laser beam severs collagen fibers of the vitreous material.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 illustrates a perspective view of an exemplary surgical system according to aspects of the present disclosure;
FIG. 2 illustrates a perspective view of an exemplary probe according to aspects of the present disclosure;
FIG. 3 is a cross-sectional illustration of an exemplary probe during use with a patient according to aspects of the present disclosure;
FIG. 4 is a block diagram of a vitrectomy probe system according to aspects of the present disclosure;
FIG. 5 is a stylized diagram showing a portion of the probe of FIG. 2, according to aspects of the present disclosure;
FIG. 6A is a side cross sectional view showing a portion of the probe of FIG. 2, according to aspects of the present disclosure;
FIG. 6B is a side cross sectional view of the probe taken along lines 6B-6B' in FIG. 6A, according to aspects of the present disclosure; and
FIG. 7 is a flowchart showing an illustrative method for treating a patient with a vitrectomy probe.

The accompanying drawings may be better understood by reference to the following detailed description.

### DETAILED DESCRIPTION

For the purposes of promoting an understanding of the principles of the present disclosure, reference will now be made to the implementations illustrated in the drawings, and specific language will be used to describe the same. It will nevertheless be understood that no limitation of the scope of the disclosure is intended unless specifically indicated. Any alterations and further modifications to the described devices, instruments, and any further application of the principles of the present disclosure are fully contemplated as would normally occur to one skilled in the art to which the disclosure relates. In particular, it is fully contemplated that the features, components, and/or steps described with respect to one implementation may be combined with the features, components, and/or steps described with respect to other implementations of the present disclosure. For simplicity, in some instances the same reference numbers are used throughout the drawings to refer to the same or like parts.

The present disclosure is broadly directed to systems and instruments for treating an eye of a patient. In one or more embodiments, a vitrectomy probe may include a body, and a disruption element extending from the body, wherein the disruption element includes a needle having a main lumen and a port at a distal end thereof. The disruption element may further include an ultraviolet (UV) optical fiber projecting a UV laser beam for irradiating an area proximate the port. In some embodiments, a UV light source is optically connected with the UV optical fiber, the UV light source generating the UV laser beam in a spectral range of approximately 190 - 220 nanometers to target collagen fibers of a vitreous material entering the port. According to some embodiments, as soon as the vitreous material is drawn into the probe, e.g., through a port, the vitreous material passes through a volume irradiated by the laser beam emitted by the UV optical fiber, thus severing the vitreous material. The removal of the vitreous material may be of particular importance, because residual vitreous material can cause post-operative retinal tearing or retinal detachment.

One or more of the following technical benefits and advantages may be achieved by cutting collagen fibers present in the vitreous material using a UV laser beam. First, the collagen fibers are directly attached to the retina, and can cause retinal detachment or damage if pulled too hard. By using a UV laser to process the collagen, the amount of tension on the retina during the medical procedure can be reduced. Second, the long collagen fibers make the vitreous material too viscous or too difficult to aspirate efficiently without being cut. By using a UV laser to process the collagen, the fibers are cut or broken down in a way to facilitate aspiration. Third, UV laser treatments allow higher repetition rates, which helps reduce the amount of material needed to be pulled into the port of the probe for each cut, further reducing tension on the retina. Fourth, a higher repetition rate may reduce the vacuum level required to aspirate the vitreous material. Fifth, by using a UV laser to process the collagen fibers, interruption of the aspiration flow for the cutting blade can be avoided, thus reducing pulsations on the retina.

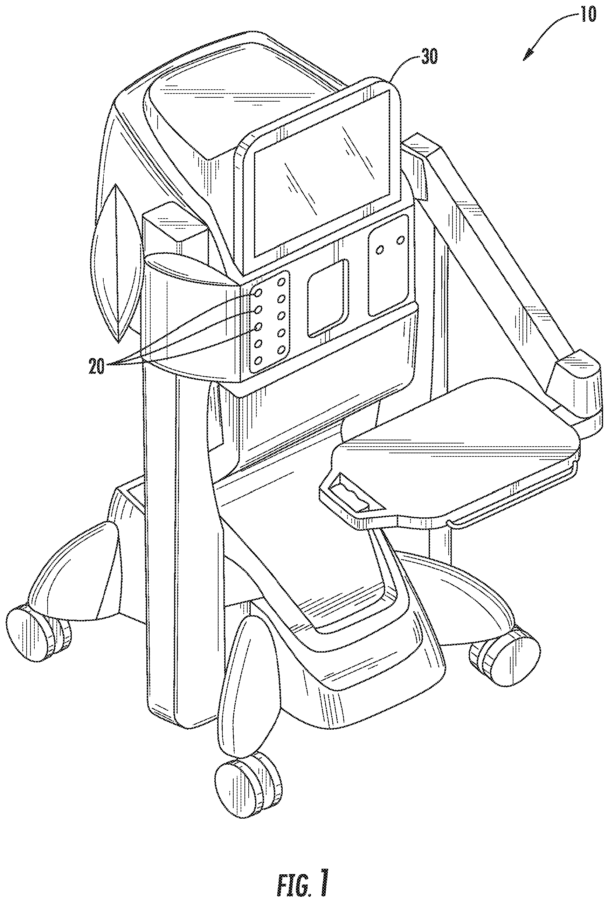

**FIG. 1** shows an example surgical console (interchangeably referred to as "console") 10 within the scope of the present disclosure. The console 10 may be a vitreoretinal surgical console, such as the Constellation^{®} surgical console produced by Alcon Laboratories, Inc., 6201 South Freeway, Fort Worth, TX, 76134. As shown, the console 10 may include one or more ports 20. The ports 20 may be utilized for providing infusion and/or irrigation fluids to the eye or for aspirating materials from the eye. The console 10 may also include a display 30 for interfacing with the console 10, such as to establish or change one or more operations of the console 10. In some instances, the display 30 may include a touch-sensitive screen for interacting with the console 10 by touching the screen of the display 30. A probe, such as a vitrectomy probe, may be coupled to a port 20 for dissecting ocular tissues and aspirating the ocular tissues from the eye.

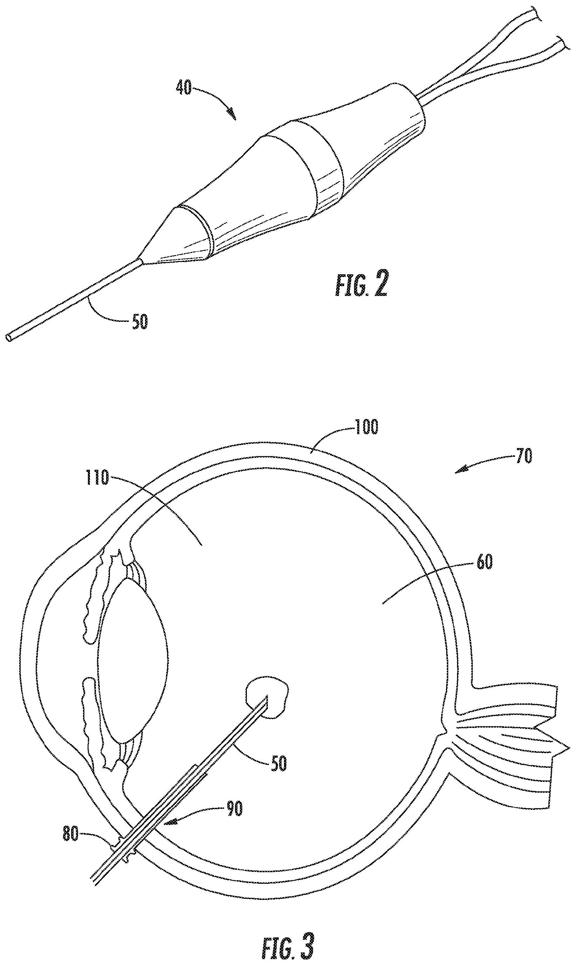

**FIG. 2** shows an example vitrectomy probe 40 (hereinafter "probe"). The probe 40 includes a laser cutter 50. As illustrated in **FIG. 3****,** during an ophthalmic surgical procedure, such as a retinal surgical procedure, the laser cutter 50 may be inserted into the posterior segment 60 of the eye 70, such as through a cannula 80 disposed in an incision 90 through the sclera 100 of the eye 70, to remove and aspirate ocular tissues. For example, during a retinal surgical procedure, the laser cutter 50 may be inserted into the posterior segment 60 of the eye 70 to remove vitreous humor (interchangeably referred to herein as "vitreous" or "vitreous material") 110, a jelly-like substance that occupies the volume defined by the posterior segment 60. The laser cutter 50 may also be used to remove membranes covering the retina or other tissues.

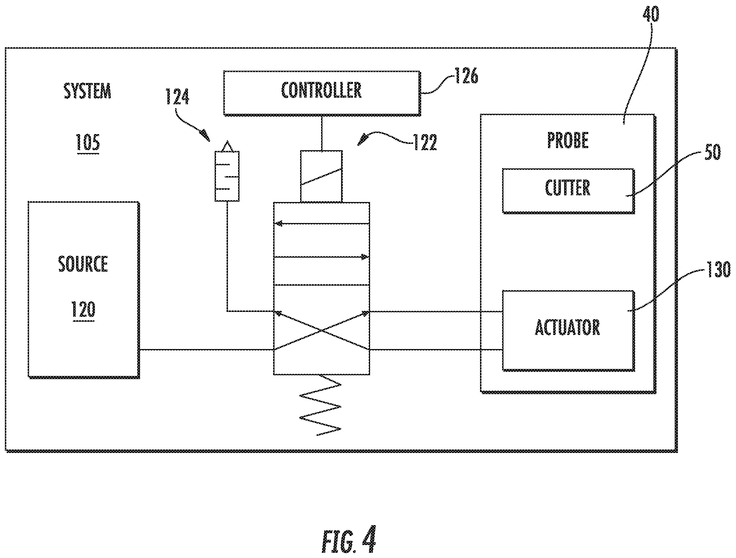

**FIG. 4** is a schematic illustration of exemplary components of a vitrectomy probe system 105. The vitrectomy probe system 105 may include the vitrectomy probe 40, a pneumatic pressure source 120, a probe driver shown as an adjustable directional on-off pneumatic driver (hereinafter "driver") 122, a muffler 124, and a controller 126. In some embodiments, the controller 126 may be a processor that includes one or more processing cores capable of performing parallel or sequential operations. Alternatively, the controller 126 may be a dedicated piece of hardware such as an application specific integrated circuit (ASIC), to name just a few examples. The pneumatic pressure source 120, the driver 122, the muffler 124, and the probe 40 are in fluid communication with each other along lines representing flow paths or flow lines. The controller 126 is in electrical communication with the driver 122. In some embodiments, the controller 126 controls operation of both the driver 122 and various aspects of the probe 40, including movement and/or the frequency of oscillation by way of the actuator 130 as well as a flow rate of fluid to/from the surgical site.

Although not shown for the sake of brevity, the vitrectomy probe system 105 may include a number of subsystems that are used together to perform ocular surgical procedures, such as emulsification or vitrectomy surgical procedures. The vitrectomy probe system 105 may include an information or data storage system, which may include one or more types of memory, such as RAM (random-access memory), ROM (read-only memory), flash memory, a disk-based hard drive, and/or a solid-state hard drive. The controller 126 and data storage system may communicate over a bus, which may also permit communication with and between one or more of the plurality of subsystems of the vitrectomy probe system 105.

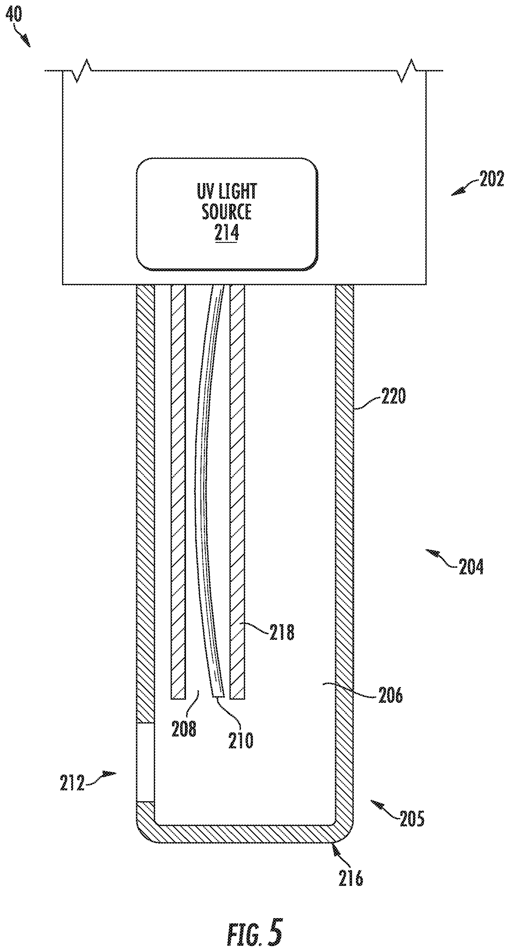

**FIG. 5** is a stylized diagram showing a portion of the illustrative vitrectomy probe 40. According to the present example, the vitrectomy probe 40 includes a body 202 and a disruption element 204 supported by and extending therefrom. The disruption element 204 includes a main lumen 206 having a port 212 at a distal tip 205. The disruption element 204 may also include a fiber lumen 208 with an optical fiber 210 therein. Alternatively, the optical fiber 210 may be provided without the fiber lumen 208. In some embodiments, the UV optical fiber 210 is a UV laser fiber, such as an air-core or photonic crystal fiber.

The body 202 forms a handle portion that may be grasped and manipulated by a surgeon when performing a surgical procedure, such as a vitrectomy. In some embodiments, the exterior portion of the body 202 is ergonomically designed for comfortable grasping by the surgeon. The body 202 may be made from a variety of materials commonly used to form such tools. For example, the body 202 may be made of a lightweight aluminum, a polymer, or other material. In various embodiments, the body 202 may be sterilized and used in more than one surgical procedure, or it may be a single-use device. The inner portion of the body 202 may be designed to house a UV light source 214. In other embodiments, the UV light source 214 is located external to the body 202. The inner portion of the body 202 also may be designed to support the disruption element 204 and other features or elements of the probe 40.

The disruption element 204 is a portion of the probe 40 that interfaces with the patient. The disruption element 204 may be designed to penetrate a globe of an eye, and may be used to remove vitreous or perform other functions or tasks. The disruption element 204 may include a needle 216, a fiber cannula 218, and the UV optical fiber 210. The needle 216 includes the distal tip 205, the main lumen 206, and a cylindrical body portion 220. The cylindrical body portion 220 may include the port 212 near the distal tip 205. In one example, the main lumen 206 has a substantially circular cross-section. In other examples, the main lumen 206 may have other cross-sectional shapes, including oval, rectangular, among others. The port 212, which is at the distal tip 205 of the needle 216, may be sized and shaped to allow vitreous fibrils to enter the main lumen 206. As will be described in further detail below, a UV laser beam projecting from the UV optical fiber 210 is operable to sever the vitreous fibrils that enter the port 212.

The fiber cannula 218 may be disposed within the main lumen 206 of the needle 216. In some embodiments, the fiber cannula 218 includes the fiber lumen 208 and is designed to house the UV optical fiber 210. The fiber cannula 218 may be rigidly fixed in place and may be secured along an interior of the needle 216, or may float within the main lumen 206. In some embodiments, the fiber cannula 218 is formed within a wall of the needle 216. In one example, the outer surface of the fiber cannula 218 is secured to the inner surface of the needle 216. In some embodiments, the interior of the fiber cannula 218 may have a diameter that is substantially larger than the outer diameter of the UV optical fiber 210 such that the UV optical fiber 210 is able to displace within the fiber lumen 208. The outer diameter of the fiber cannula 218 may be sized and shaped to fit within the main lumen 206, while leaving enough room within the interior of the main lumen 206 for other purposes, such as the aspiration of emulsified or disrupted tissue, including vitreous fibrils.

In some embodiments, the UV optical fiber 210 may be designed to operate as an optical waveguide and propagate a UV laser beam. The characteristics of the UV laser beam propagated through the UV optical fiber 210 are such that the UV laser beam causes disruption of vitreous fibrils within the path of the UV laser beam. In some examples, the UV laser beam may be produced by the UV light source 214, in the body 202, or at another location about the vitrectomy probe system 105. The UV laser beam may be in the 190-220 nm spectral range, which allows absorption of the collagen fibers to be high (e.g., > 2000 cm⁻¹), while the absorption of water, comprising approximately 99% of the vitreous, is significantly lower (e.g., < 2cm⁻¹). As a result, virtually all the optical energy delivered from the UV optical fiber 210 will go towards destroying the collagen fibers, while only a minimal amount of the optical energy may remain, thus reducing heat generation of the fluids in the eye. Furthermore, the absorption in the collagen fibers increases by a factor of 7-10 between 240 nm and 220 nm, allowing the optical fiber to disrupt the collagen in a small volume of vitreous, which limits the energy requirements of the UV light source 214 and the UV optical fiber 210.

In some embodiments, the UV laser may have a pulse rate within a range of about 10-500 kilohertz (kHz). This range can effectively provide disruption, which is the mechanical effect of light on tissue to disrupt or breakdown the tissue by laser-produced rapid ionization of molecules. Other ranges for characteristics of the UV laser beam that can provide disruption are contemplated as well.

The UV optical fiber 210 is positioned such that a UV laser beam projecting from the UV optical fiber 210 will be projected across the port 212 and has power sufficient to sever vitreous fibrils and, in particular, the collagen fibers of the vitreous. Thus, the UV laser beam can sever vitreous fibrils that enter the port 212. In the embodiments disclosed herein, the width of the UV laser beam may be substantially smaller than the width of the port 212. For example, the port 212 may have a diameter or width of approximately 300 microns. The UV laser beam itself may have a diameter of approximately 10 to 25 microns. However, embodiments herein are not limited to these dimensions. In some embodiments, the UV light source 214 may cause the UV laser beam being projected from the UV optical fiber 210 to scan across the port 212.

In some embodiments, the actuator 130 (**FIG**. **4**) may be configured to mechanically move the UV optical fiber 210 within the fiber lumen 208. Thus, the size of the fiber lumen 208 and size of the UV optical fiber 210 may be such that there is room for the UV optical fiber 210 to physically move within the fiber lumen 208. Specifically, the diameter of the fiber lumen 208 may be larger than the outer diameter of the UV optical fiber 210.

In some embodiments, the UV light source 214 includes a motor or driver that displaces the UV optical fiber 210. Furthermore, the UV light source 214 may move the UV optical fiber 210 in a variety of different ways at varying frequencies. For example, the UV light source 214 may move the UV optical fiber 210 at a frequency within a range of about 10 hertz (Hz) to 10 kHz. This rapid movement of the UV optical fiber 210 causes the UV laser beam being projected from the UV optical fiber 210 to move across the port 212 fast enough so that vitreous fibrils entering the port 212 will be within the path of the UV laser beam. In various embodiments, the UV light source 214 may displace the UV optical fiber 210 in a back and forth or side-to-side motion, a circular rotation, a random path, or other displacement pathway. The UV laser beam can then sever the vitreous fibrils through a disruption process.

In some non-limiting embodiments, the UV optical fiber 210 may be coupled to one or more illumination sources. Example illumination sources may include the UV light source 214, an infrared ("IR") source, or other desired light or radiation source. While "light" is discussed herein, the scope of the disclosure is not intended to be limited to visible light. On the contrary and as indicated above, other types of radiation, such as UV and IR radiation, may be transmitted through and emitted from one or more of the UV optical fibers 210. The term "light" is intended to encompass any type of radiation for use with the UV optical fiber 210. Further, in some instances, the UV optical fibers 210 may be multi-mode end-emitting fibers. However, in other implementations, other types of light-emitting optical fibers may be used.

UV light from the UV light source 214 may be conveyed through the distal tip of the UV optical fiber 210. As explained above, the end of the UV optical fiber 210 at the distal tip defines the illumination aperture. In some implementations, the UV optical fiber 210 may have a diameter in the range of 25 µm to 75 µm. In some particular implementations, the UV optical fiber 210 may have a diameter within the range of about 40 µm to 50 µm. In still other implementations, the UV optical fiber 210 may have a diameter that is larger or smaller than the diameters described. For example, some deep UV silica fibers may be greater than 100 µm in diameter. In some implementations, a light sleeve assembly may have a plurality of optical fibers 210 that are different or the same size.

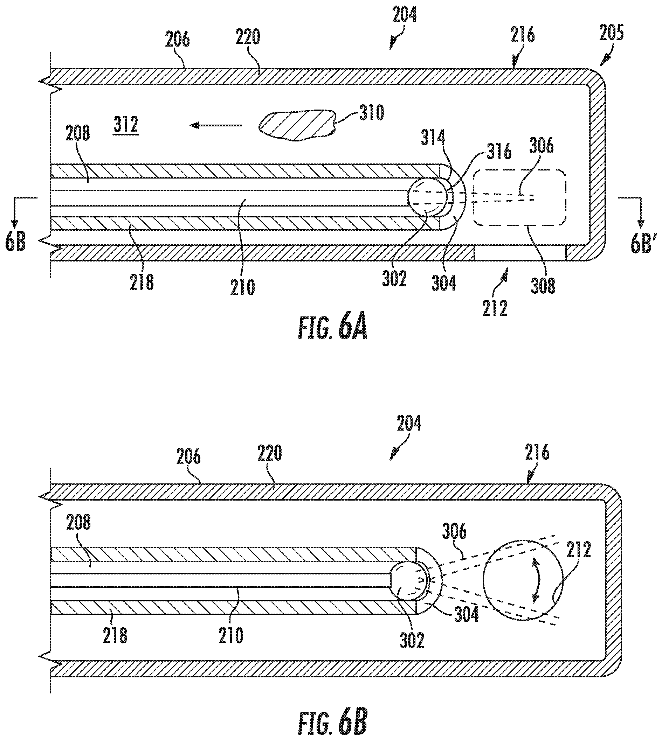

**FIGS. 6A** and **6B** are diagrams showing longitudinal cross-sectional views of the disruption element 204 of the vitrectomy probe 40 with the UV optical fiber 210. **FIG. 6A** illustrates a view along a cross-section that is perpendicular and through the port 212. **FIG. 6B** illustrates a view taken along lines 6B-6B' in **FIG. 6A****,** showing a cross-section that is parallel to the port 212.

In the example of **FIG. 6A****,** the tip of the UV optical fiber 210 may include a rounded tip functioning as a lens. The tip will thus be referred to as a lensed tip 302. In other embodiments, no lensed tip is present. The lensed tip 302 may be a solid, round end of the UV optical fiber 210. The lensed tip 302 may have a diameter that is larger than the diameter of the UV optical fiber 210. The lensed tip 302 may be made of the same material as the UV optical fiber 210 and thus be transparent. The lensed tip 302 may function independently, or in conjunction with a lens 304, to provide refractive focusing power. This can provide a means to concentrate the energy of the projected laser beam by reducing the divergence of the beam, collimating the beam, or converging the beam, for example to a spot smaller than the UV optical fiber diameter.

In some examples, the lensed tip 302 may also function as a bearing in conjunction with a lens 304. The lensed tip 302 or bearing is sized and shaped to fit within the lens 304, which is secured to the distal end of the fiber lumen 208. The lens 304 may be made of a transparent material such as glass or plastic. The lens 304 may have a concave inner surface that receives the bearing, while the outer surface of the lens 304 may have a convex shape. The curvature of both the outer surface and the inner surface may be selected to affect the UV laser beam 306 as desired. For example, the curvature of both surfaces of the lens 304 can cause the laser beam to be collimated, convergent, or divergent. In some examples there may be a lubricant 314 between the bearing and the lens 304. The lubricant 314 may be a transparent fluid that has a refractive index that matches the refractive index of the material that forms the lens 304. This reduces the amount of reflection of the UV laser beam 306 being projected from the UV optical fiber 210. In other embodiments, a different kind of lubricant may be used. In yet other embodiments, no lubricant is used between the bearing and the lens 304.

The actuator (e.g. 130, FIG. 4) may cause movement of the UV optical fiber 210 such that the bearing rotates or spins within the lens 304 as the UV optical fiber 210 moves within the fiber lumen 208. As the bearing rotates with respect to the lens 304, the distal end 316 of the UV optical fiber 210, through which a UV laser beam 306 is emitted, moves such that the UV laser beam 306 being projected from the distal end 316 of the UV optical fiber scans across the port 212. In other embodiments, the UV laser beam 306 does not move and, instead, is stationary as the vitreous is suctioned past the UV laser beam 306.

In some embodiments, the UV optical fiber 210 may move in a variety of ways. For example, the UV optical fiber 210 may move in an elliptical or circular motion around the inner diameter of the fiber lumen 208. In some examples, the UV optical fiber 210 may move back and forth along a linear path across the fiber lumen 208. In some cases, the UV optical fiber 210 may move at random throughout the fiber lumen 208.

During operation of the vitrectomy probe 40, the surgeon may move the tip of the vitrectomy probe 40 such that vitreous fibrils enter into the port 212. As the vitreous fibrils enter the port 212 and pass into the disruption area or region 308, they will be severed as the scanning UV laser beam 306 moves past them and/or as they pass through the UV laser beam 306. By using the UV light source 214, e.g., in the 190-220 nm spectral range, a lower optical pulse energy may be selected, while still efficiently targeting the collagen chains that cause the vitreous to be difficult to extract.

In some embodiments, the UV light source 214 may operate in the range of 1-10 ns (e.g. 193 nm ArF Excimer, 213 nm Nd:YAG 5^{th} harmonic), which is generally fast enough to cause localized damage to the absorbing material without heating up a larger area via thermal diffusion. In other embodiments, pulse lengths in the femtosecond range (<1000 fs) may be used at longer wavelengths. The peak intensities of these ultra-short pulses is high enough that a direct absorption feature is not required for the laser to interact with matter. Instead, the UV laser beam 306 works using photodisruption in which the large electric field produced by a focused pulse strips electrons off of the atoms and produces a plasma, thus destroying the material at the focus of the UV laser beam 306. This principle is used in various systems to cut the cornea and lens of an eye, for example, during a cataract procedure. In some examples, using light at ~1030 is not absorbed as it passes through the transparent tissue. Only at the focus of the UV laser beam 306, does the high field cause photodisruption, which allows cuts to be made behind or inside the transparent tissue without harming the surface tissue.

As further shown, in some examples, the vitrectomy probe 40 includes an aspiration lumen 312 for aspirating the severed vitreous material 310 and other vitreous fluids. The aspiration lumen 312 may be in connection with a suction mechanism (not shown) that provides a vacuum force to extract the severed vitreous material 310 and other fluids. In other examples, the main lumen 206 acts as part of the aspiration lumen 312, as illustrated. In some examples, however, a separate and independent cannula with an aspiration lumen is positioned within the main lumen 206. Such an aspiration lumen is in connection with the port 212 so that severed vitreous material 310 will appropriately pass into the aspiration lumen.

**FIG. 6B** illustrates a cross-sectional view of the disruption element 204 taken along lines 6B-6B' in **FIG. 6A****.** Thus, it can be seen that the UV laser beam 306 scans across the circular port 212 as indicated by the double-sided arrow. While the port 212 is illustrated as circular, it is understood that the port 212 may have other shapes, including elliptical or rectangular, for example.

Various repetition rates, focused spot diameters, spot densities, scanned areas, and scan patterns can be used in accordance with principles described herein. For example, a 30 kHz laser pulse rate with a focused beam diameter of 3 microns applied at 100% density over a 500 micron diameter port could be used in accordance with an agitation technique that involves a 50 Hz oscillation in an elliptical pattern. In another example, a 200 kHz laser pulse rate with a focused beam diameter of 10 microns applied at 50% density over a 300 micron diameter circular area could be used in accordance with a 4500 Hz oscillation in an elliptical path.

In some examples, the UV laser beam 306 is configured such that it converges as it crosses the port 212. A converging laser beam has a diameter that decreases over a specific length. A converging laser beam focuses more energy into a smaller cross-sectional area, thus allowing for better photo-disruption at the smaller area.

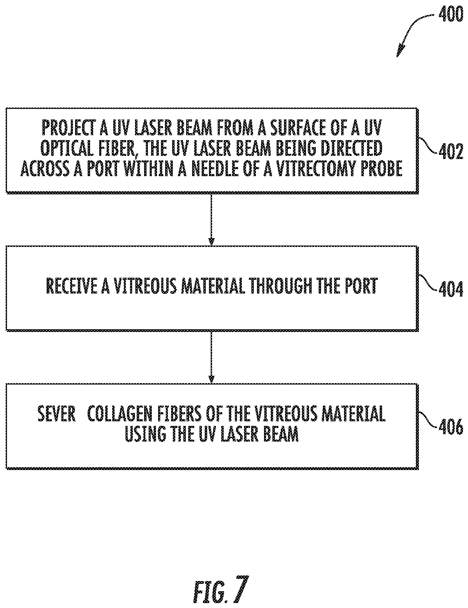

**FIG. 7** is a diagram illustrating a method for treating a patient using a vitrectomy probe. As shown, at block 402 the method 400 not forming part of this invention may include projecting a UV laser beam from a UV optical fiber, the UV laser beam directed across a port of a needle of a vitrectomy probe. In some embodiments, the UV optical fiber is housed within a fiber cannula, which is within the needle, the fiber cannula having a fiber lumen. In some embodiments, the main lumen is an aspiration lumen for extracting a vitreous material that is severed by the UV laser beam projected from the UV optical fiber. In some embodiments, the UV laser beam may be generated in a spectral range of approximately 190 - 220 nanometers. In some embodiments, the UV laser beam may be a pulsed laser beam. In some embodiments, a pulse of the laser beam may have a width within a range of about 10-1000 femtoseconds (fs).

At block 404, the method 400 may include receiving a vitreous material through the port. At block 406, the method 400 may include severing collagen fibers of the vitreous material using the UV laser beam. In some embodiments, the UV laser beam is directed towards the distal end of the needle to cut the collagen fiber. In some embodiments, the UV optical fiber is configured to emit the laser beam in a converging pattern across the port.

In sum, the UV laser beam vitrectomy probe described herein allows the collagen fibers of vitreous material to be easily removed, which reduces the retinal traction produced by removing the vitreous material. Also, using an appropriate UV laser, e.g., in a spectral range between 190 - 220 nanometers, permits the use of lower optical pulse energy, as the collagen chains/fibers that cause the vitreous to be difficult to extract are efficiently targeted.

Some embodiments may be described using the expressions "proximal" and "distal" when used in connection with UV vitrectomy probes. As used herein, "proximal" refers to the end of the probe closest to the medical operator, whereas "distal" refers to the end of the probe inserted into a patient. Furthermore, as used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

Although specific embodiments have been illustrated and described herein, it should be appreciated that any arrangement calculated to achieve the same purpose may be substituted for the specific embodiments shown. This disclosure is intended to cover any and all adaptations or variations of various embodiments. It is to be understood that the above description has been made in an illustrative fashion, and not a restrictive one. Combinations of the above embodiments, and other embodiments not specifically described herein will be apparent to those of skill in the art upon reviewing the above description. Thus, the scope of various embodiments includes any other applications in which the above compositions, structures, and methods are used.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A vitrectomy probe system (105) for treating an eye of a patient, the vitrectomy probe (40) comprising:
a body (202); and
a disruption element (204) extending from the body, the disruption element comprising:
a needle (216) comprising a main lumen and a side port (212) at a distal end of the needle;
an ultraviolet (UV) optical fiber (210), the UV optical fiber projecting a UV laser beam for irradiating an area proximate the port;
an actuator (130) for controlling movement of the UV optical fiber; and
wherein the system further comprises:
a UV light source (214) for generating the UV laser beam in a spectral range of approximately 190 - 220 nanometers;
wherein the UV optical fiber (210) is mechanically moved within the fiber lumen (208) such that the UV laser beam being projected from the UV optical fiber (210) moves across the side port to sever vitreous.

2. The vitrectomy probe system of claim 1, wherein the main lumen (206) is an aspiration lumen (206, 312) for extracting vitreous material that is severed by the UV laser beam projected from the UV optical fiber (210).

3. The vitrectomy probe system of claim 2, wherein the UV laser beam from the UV optical fiber (210) is projected between the port (212) and the aspiration lumen (206, 312).

4. The vitrectomy probe system of claim 1, wherein the UV optical fiber (210) comprises a rounded tip operable as a lens (302).

5. The vitrectomy probe system of claim 1, wherein the UV laser beam (306) is directed towards the distal end of the needle (216) to cut collagen fibers of a vitreous material entering the port (212).

6. The vitrectomy probe system of claim 1, wherein the UV optical fiber (210) emits the UV laser beam in a converging pattern across the port (212).

7. The vitrectomy probe of claim 1, wherein the UV optical fiber (210) is configured to move in a back and forth, side to side, or in a circular rotation.

## Patentansprüche

1. Vitrektomiesondensystem (105) zum Behandeln eines Auges eines Patienten, wobei die Vitrektomiesonde (40) Folgendes umfasst:
einen Hauptteil (202); und
ein Zerrüttungselement (204), das sich von dem Hauptteil erstreckt, wobei das Zerrüttungselement Folgendes umfasst:
eine Nadel (216), die ein Hauptlumen und einen Seitenport (212) an einem distalen Ende der Nadel umfasst;
eine Ultraviolett-Lichtleitfaser bzw. UV-Lichtleitfaser (210), wobei die UV-Lichtleitfaser einen UV-Laserstrahl zum Bestrahlen eines dem Port proximalen Gebiets projiziert;
einen Aktor (130) zum Steuern von Bewegung der UV-Lichtleitfaser; und wobei des System ferner umfasst:
eine UV-Lichtquelle (214) zum Erzeugen des UV-Laserstrahls in einem Spektralbereich von ungefähr 190-220 nm;
wobei die UV-Lichtleitfaser (210) innerhalb des Faserlumens (208) derart mechanisch bewegt wird, dass sich der von der UV-Lichtleitfaser (210) projizierte UV-Laserstrahl über den Seitenport hinwegbewegt, um den Glaskörper zu zertrennen.

2. Vitrektomiesondensystem nach Anspruch 1, wobei das Hauptlumen (206) ein Aspirationslumen (206, 312) zum Extrahieren von Glaskörpermaterial, das durch den von der UV-Lichtleitfaser (210) projizierten UV-Laserstrahl zertrennt wurde, ist.

3. Vitrektomiesondensystem nach Anspruch 2, wobei der UV-Laserstrahl aus der UV-Lichtleitfaser (210) zwischen den Port (212) und das Aspirationslumen (206, 312) projiziert wird.

4. Vitrektomiesondensystem nach Anspruch 1, wobei die UV-Lichtleitfaser (210) eine verrundete Spitze, die als eine Linse (302) betreibbar ist, umfasst.

5. Vitrektomiesondensystem nach Anspruch 1, wobei der UV-Laserstrahl (306) zum distalen Ende der Nadel (216) gerichtet ist, um Kollagenfasern eines Glaskörpermaterials, das in den Port (212) eintritt, zu schneiden.

6. Vitrektomiesondensystem nach Anspruch 1, wobei die UV-Lichtleitfaser (210) den UV-Laserstrahl in einem konvergierenden Muster über den Port (212) hinweg emittiert.

7. Vitrektomiesonde nach Anspruch 1, wobei die UV-Lichtleitfaser (210) ausgelegt ist zum Bewegen in einer Vor- und Zurück-, einer von Seite-zu-Seite- oder in einer kreisförmigen Rotation.

## Revendications

1. Système de sonde de vitrectomie (105) destiné à traiter un œil d'un patient, la sonde de vitrectomie (40) comprenant :
un corps (202) ; et
un élément de fractionnement (204) s'étendant depuis le corps, l'élément de fractionnement comprenant :
une aiguille (216) comprenant une lumière principale et un orifice latéral (212) à une extrémité distale de l'aiguille ;
une fibre optique ultraviolette (UV) (210), la fibre optique UV projetant un faisceau laser UV pour irradier une zone proche de l'orifice ;
un actionneur (130) pour contrôler le déplacement de la fibre optique UV ; et
le système comprenant en outre :
une source de rayonnement UV (214) pour générer le faisceau laser UV dans un domaine spectral d'environ 190-220 nanomètres ;
dans lequel la fibre optique UV (210) est déplacée mécaniquement à l'intérieur de la lumière de fibre (208) de telle sorte que le faisceau laser UV projeté depuis la fibre optique UV (210) se déplace à travers l'orifice latéral pour sectionner le corps vitré.

2. Système de sonde de vitrectomie selon la revendication 1, dans lequel la lumière principale (206) est une lumière d'aspiration (206, 312) destinée à extraire la matière vitreuse qui est sectionnée par le faisceau laser UV projeté depuis la fibre optique UV (210).

3. Système de sonde de vitrectomie selon la revendication 2, dans lequel le faisceau laser UV issu de la fibre optique UV (210) est projeté entre l'orifice (212) et la lumière d'aspiration (206, 312).

4. Système de sonde de vitrectomie selon la revendication 1, dans lequel la fibre optique UV (210) comprend une pointe arrondie utilisable comme une lentille (302).

5. Système de sonde de vitrectomie selon la revendication 1, dans lequel le faisceau laser UV (306) est dirigé vers l'extrémité distale de l'aiguille (216) pour couper les fibres de collagène d'une matière vitreuse entrant dans l'orifice (212).

6. Système de sonde de vitrectomie selon la revendication 1, dans lequel la fibre optique UV (210) émet le faisceau laser UV en un motif convergent à travers l'orifice (212) .

7. Sonde de vitrectomie selon la revendication 1, dans laquelle la fibre optique UV (210) est configurée pour se déplacer d'avant en arrière, d'un côté à l'autre, ou dans une rotation circulaire.
